(19) Europäisches Patentamt
European Patent Office
Office européen des brevets

(11) **EP 2 564 189 B1**

(12) **EUROPÄISCHE PATENTSCHRIFT**

(45) Veröffentlichungstag und Bekanntmachung des Hinweises auf die Patenterteilung:
**16.07.2014 Patentblatt 2014/29**

(21) Anmeldenummer: **11710662.5**

(22) Anmeldetag: **18.03.2011**

(51) Int Cl.:
*G01N 33/36* *(2006.01)*        *D01H 13/22* *(2006.01)*

(86) Internationale Anmeldenummer:
**PCT/CH2011/000056**

(87) Internationale Veröffentlichungsnummer:
**WO 2011/134091 (03.11.2011 Gazette 2011/44)**

(54) **PRÜFUNG VON REGELMÄSSIGEN SOLLWERTABWEICHUNGEN EINES PARAMETERS IN EINEM LÄNGLICHEN TEXTILEN PRÜFGUT**

TESTING FOR REGULAR SETPOINT VALUE DEVIATIONS OF A PARAMETER IN AN ELONGATE TEXTILE TEST OBJECT

CONTRÔLE DE VARIATIONS RÉGULIÈRES DE LA VALEUR THÉORIQUE D'UN PARAMÈTRE DANS UNE MATIÈRE D'ESSAI TEXTILE ALLONGÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.04.2010 CH 622102010**

(43) Veröffentlichungstag der Anmeldung:
**06.03.2013 Patentblatt 2013/10**

(73) Patentinhaber: **Uster Technologies AG 8610 Uster (CH)**

(72) Erfinder:
• GALLIKER, Christoph
  CH-8004 Zürich (CH)
• SCHMID, Peter
  CH-8050 Zürich (CH)

(56) Entgegenhaltungen:
**EP-A1- 0 685 580      WO-A2-2007/056883
DE-A1- 3 928 417**

**Beschreibung**

FACHGEBIET

Die vorliegende Erfindung liegt auf dem Gebiet der textilen Qualitätskontrolle. Sie betrifft ein Verfahren und eine Vorrichtung zur Prüfung von regelmässigen Ereignissen in einem länglichen textilen Prüfgut, gemäss den Oberbegriffen der unabhängigen Patentansprüche. Derartige Verfahren und Vorrichtungen kommen vorzugsweise in Garnreinigern auf Spinn- oder Spulmaschinen zum Einsatz.

Begriffe wie "regelmässige Ereignisse", "Regelmässigkeit" etc., werden dieser Schrift im Wesentlichen synonym zu "periodische Ereignisse" bzw. "Periodizität" verwendet. Die ersteren Begriffe werden deshalb vorgezogen, weil sie etwas mehr Spielraum offen lassen als die mathematisch strenge "Periodizität" und damit für die textile Praxis tauglicher sind.

STAND DER TECHNIK

Zur Sicherung der Garnqualität werden an Spinn- oder Spulmaschinen so genannte Garnreiniger eingesetzt. Eine derartige Vorrichtung ist z. B. aus der US-5,537,811 A bekannt. Sie beinhaltet einen Messkopf mit mindestens einem Sensor, der das bewegte Garn abtastet. Häufig verwendete Sensorprinzipien sind das kapazitive (siehe z. B. EP-0'924'513 A1) oder das optische (siehe z. B. WO-93/13407 A1). Ziel der Abtastung ist es, Fehlstellen wie Dickstellen, Dünnstellen oder Fremdstoffe im Garn zu detektieren. Das Ausgangssignal des Sensors wird laufend mit vorgegebenen Bewertungskriterien wie z. B. einer Reinigungsgrenze bewertet. Ein Beispiel für eine Reinigungsgrenze ist in der US-5,537,811 A angegeben. Liegt eine Fehlstelle unterhalb der Reinigungsgrenze, so ist sie tolerierbar; liegt sie oberhalb der Reinigungsgrenze, so ist sie nicht tolerierbar und wird aus dem Garn entfernt oder einfach registriert. Die Fehlstellen können in einem zweidimensionalen Klassierschema, in dem üblicherweise entlang der Abszisse die Fehlerlänge und entlang der Ordinate die Fehlergrösse (Abweichung der Masse pro Garnlänge, des Garnquerschnitts, der Garnreflektivität etc. von einem Sollwert) aufgetragen sind. Beispiele für solche Klassierschemata sind in der US-5,537,811 A und im Absatz 3.2.4 "Classification values" des USTER® *QUANTUM 2* Application Manual Winding, Uster Technologies AG, November 2006, beschrieben.

Es gibt allerdings Garnfehler, die auch dann störend sind, wenn sie unterhalb der Reinigungsgrenze liegen: die periodischen Garnfehler. Dies sind Dick- oder Dünnstellen, die in immer gleichem Abstand auftreten. Periodische Garnfehler sind unerwünscht, weil sie zu Moiré-Effekten in Gewebe oder Gestrick führen können. Sie werden im Spinnprozess erzeugt, wenn fadenführende Elemente defekt sind, bspw. durch einen unrunden Vorderzylinder einer Ringspinnmaschine.

Seit es elektronische Garnreiniger gibt, wurde versucht, damit auch periodische Garnfehler zu erfassen. Dies einerseits, um die Garnqualität durch Entfernung der periodischen Garnfehler zu erhöhen, und andererseits, um Rückschlüsse auf das defekte Element zu erhalten, welches die unerwünschte Periode erzeugt. Ein frühes Beispiel dafür liefert die US-2,950,435 A. Diese Schrift sowie auch neuere Schriften wie die US-5,592,849 A oder die EP-2'090'538 A2 transformieren das zeitabhängige (bzw. ortsabhängige) Ausgangssignal des Garnreinigers mittels Fourier-Transformation in den Zeitfrequenzraum (bzw. Ortsfrequenzraum). Die im Zeitfrequenzraum (bzw. Ortsfrequenzraum) mit grossen Amplituden vertretenen Frequenzen deuten auf periodische Garnfehler hin, während die Amplituden der übrigen Frequenzen ein Mass für die aperiodische Garnungleichmässigkeit sind. Gemäss der DE-39'28'417 A1 werden sowohl eine Klassierung der Garnfehler als auch eine Auffindung von regelmässigen Garnfehlern mittels Fourier-Transformation unabhängig voneinander durchgeführt. Solche Verfahren, die mit der Fourier-Transormation arbeiten, ermöglichen zwar eine nachträgliche Aussage über das Vorhandensein von periodischen Garnfehlern, aber nicht deren Entfernung während des Herstellungsprozesses (online).

Der Garnreiniger USTER® *QUANTUM 2* der Anmelderin beschreitet einen anderen Weg, der im Kapitel 6 "Periodic yarn faults" des USTER® QUANTUM 2 Application Manual Winding, Uster Technologies AG, November 2006, beschrieben ist. Er verlässt den Zeitraum (bzw. Ortsraum) nicht und erlaubt es, periodische Garnfehler, die "Pearl Chains" genannt werden, online aus dem Garn zu entfernen. Der Garnreiniger hat dafür einen so genannten Pearl-Chain-Kanal. Damit im Pearl-Chain-Kanal ein Garnschnitt erfolgt, müssen die folgenden vier Kriterien erfüllt sein:

- Empfindlichkeit in % = minimale Garnfehlergrösse,
- Länge in cm = minimale Garnfehlerlänge,
- Fehlerabstand in cm = Abstand von Garnfehler zu Garnfehler, und
- Anzahl Fehler = Fehleranzahl, die zum Schnitt führt.

Die vier jeweils rechts vom Gleichheitszeichen stehenden festen Schwellwerte müssen vom Benutzer vorgegeben werden. Dieses Verfahren wirft, bildhaft gesprochen, alle periodischen Garnfehler in denselben Topf, der nur durch die minimale Garnfehlergrösse und die minimale Garnfehlerlänge im Klassierschema begrenzt ist. Es erlaubt keine Unterscheidung zwischen unterschiedlichen periodischen Garnfehlern. Ausserdem setzt es Kenntnisse über die gesuchten periodischen Garnfehler, z. B. den Fehlerabstand, voraus, die jedoch nicht immer vorhanden sind.

Die WO-2007/056883 A2 offenbart ein Verfahren zur Charakterisierung von Effektgarn. Effektgarn ist im Wesentlichen ein Garn mit einer Abfolge von absichtlich erzeugten Dickstellen - so genannten Effekten - und dazwischen liegenden Stegen. Die Abfolge braucht nicht regelmässig zu sein; jedenfalls wird strenge Periodizität vermieden, weil sie zu einem Moiré-Effekt im Gewebe führt. Gemäss einer Ausführungsform der WO-2007/056883 A2 können die entlang des Effektgarns aufgenommenen Messwerte aufgeteilt werden in eine erste, idealisierte Kurve, welche nur die Effekte darstellt, und eine zweite Kurve, welche nur das Grundgarn ohne Effekte darstellt. Die beiden Kurven werden Fourier-transformiert, wodurch ein erstes Spektrogramm für die Effekte allein und ein zweites Spektrogramm für das Grundgarn erzeugt werden. Das Verfahren wird auf Stichproben im Garnlabor angewendet und eignet sich zur nachträglichen Lokalisierung von Störungen in der Effekterzeugung oder von Fehlern im Spinnprozess. Zur Online-Prüfung von regelmässigen Ereignissen, etwa bei der Garnreinigung auf Spulmaschinen, ist es jedoch ungeeignet.

## DARSTELLUNG DER ERFINDUNG

Es ist eine Aufgabe der vorliegenden Erfindung, ein Verfahren und eine Vorrichtung zur Prüfung von regelmässigen Ereignissen in einem länglichen textilen Prüfgut anzugeben, welche Nachteile des Stands der Technik vermeiden. Insbesondere soll eine differenziertere Behandlung der regelmässigen Ereignisse ermöglicht werden, wobei möglichst wenige Vorkenntnisse über die gesuchten regelmässigen Ereignisse erforderlich sein sollen. Das Verfahren und die Vorrichtung sollen zu einer Online-Prüfung von regelmässigen Ereignissen, etwa bei der Gamreinigung auf Spulmaschinen, geeignet sein.

Diese und andere Aufgaben werden durch das erfindungsgemässe Verfahren und die erfindungsgemässe Vorrichtung, wie sie in den unabhängigen Patentansprüchen definiert sind, gelöst. Vorteilhafte Ausführungsformen sind in den abhängigen Patentansprüchen angegeben.

Die Erfindung löst die Aufgabe, indem sie unterschiedliche Eigenschaften wie Länge und/oder Grösse der Ereignisse berücksichtigt. Erfindungsgemäss werden nur "ähnliche" Ereignisse in Bezug auf ein regelmässiges Auftreten untersucht. Zu diesem Zweck werden die Ereignisse in mehrere unterschiedliche Mengen eingeteilt, von denen jede Menge Ereignisse mit ähnlichen Eigenschaften enthält. Dies wird dadurch erreicht, dass die festgestellten Ereignisse zusammenhängenden Punktmengen in einem Parameterraum, der durch mindestens einen Parameter des Prüfguts aufgespannt wird, zugeordnet werden. In den verschiedenen Ereignismengen werden separate statistische Untersuchungen durchgeführt. Die Untersuchungen erfolgen vorzugsweise ausschliesslich im Ortsraum oder im Zeitraum.

Es kann also gesagt werden, dass die vorliegende Erfindung einzelne Ereignisse im Prüfgut identifiziert und betrachtet. Ein festgestelltes Ereignis wird einer Menge von ähnlichen Ereignissen zugeordnet. Die ähnlichen Ereignisse innerhalb der einzelnen, voneinander verschiedenen Ereignismengen werden in Bezug auf ein regelmässiges Auftreten statistisch untersucht. Diese Fokussierung auf die einzelnen Ereignisse ermöglicht eine schnelle Online-Prüfung des Prüfguts während eines Produktions- oder Verarbeitungsprozesses. Im Gegensatz dazu werden im Stand der Technik, z. B. in der WO-2007/056883 A2, längere Messkurven betrachtet, die viele verschiedene Ereignisse beinhalten. Die Messkurven als ganze werden Operationen wie Filterungen und Fourier-Transformationen unterzogen. Aus den Resultaten dieser Operationen werden im Stand der Technik regelmässige Ereignisse identifiziert.

Der Begriff "zusammenhängend" kann hier durchaus im Sinne der mathematischen Topologie verstanden werden. Dabei dürfte es in der Praxis keine Rolle spielen, ob der allgemeine Zusammenhang oder der speziellere Wegzusammenhang zur Definition verwendet wird. Die Punktmengen brauchen aber nicht einfach zusammenhängend zu sein.

Dementsprechend werden im erfindungsgemässen Verfahren zur Prüfung von regelmässigen Ereignissen in einem länglichen textilen Prüfgut Messwerte einer Eigenschaft des Prüfguts entlang der Längsrichtung des textilen Prüfguts erfasst. Werte mindestens eines Parameters des Prüfguts werden aus den Messwerten ermittelt. Es werden mindestens zwei verschiedene, zusammenhängende Punktmengen in einem Parameterraum, der durch den mindestens einen Parameter aufgespannt wird, definiert. Mindestens zwei verschiedene, mehr als ein Ereignis enthaltende Mengen von Ereignissen im Prüfgut mit von einem Sollwert abweichenden Parameterwerten werden gebildet, indem im Prüfgut festgestellte Ereignisse gemäss ihren Parameterwerten einer der Punktmengen zugeordnet werden. Die Ereignisse innerhalb der einzelnen, voneinander verschiedenen Mengen von Ereignissen werden in Bezug auf ein regelmässiges Auftreten separat statistisch untersucht. Resultate der statistischen Untersuchungen werden für jede der mindestens zwei Mengen von Ereignissen separat ausgegeben

Die Punktmengen werden vorzugsweise als zusammenhängende Flächen in einem zweidimensionalen Ereignisfeld definiert. Der Parameterraum kann z. B. durch eine Querdimension des Prüfguts, durch eine Masse pro Längeneinheit des Prüfguts und/oder durch eine Länge von Ereignissen in der Längsrichtung aufgespannt werden. Das Ereignisfeld kann einen Quadranten oder einen Teil eines Quadranten eines zweidimensionalen kartesischen Koordinatensystems beinhalten, dessen Abszisse eine Länge von Ereignissen in der Längsrichtung und dessen Ordinate eine Querdimension oder eine Masse pro Längeneinheit des Prüfguts angibt. In einer bevorzugten Ausführungsform sind die Punktmengen rechteckige Klassen für Ereignisse, die durch eine Unterteilung zumindest eines Teils des Ereignisfeldes durch waagrechte Klassengrenzen und senkrechte Klassengrenzen gebildet werden. Solche Klassen sind aus dem Stand der Technik bekannt, z. B. aus Absatz 3.2.4 "Classification values" des USTER® QUANTUM 2 Application Manual Winding, Uster Technologies AG, November 2006, oder aus der US-5,537,811 A. Sie wurden bisher für die quantitative Klassierung von Garnfehlern verwendet, insbesondere im Klassiersystem CLASSIMAT® der Anmelderin. Eine erfindungsgemässe Menge kann mit genau einer CLASSIMAT®-Klasse identisch sein oder aus einer Zusammenlegung mehrerer solcher Klassen bestehen.

Es ist vorteilhaft, wenn die statistischen Untersuchungen ausschliesslich im Ortsraum oder im Zeitraum erfolgen. Dies ermöglicht eine schnelle Behandlung der grossen Menge anfallender Daten und somit eine Online-Prüfung von regelmässigen Ereignissen während der Erzeugung oder Bearbeitung des Prüfguts. Somit braucht keine Fourier-Transformation in den Ortsfrequenzraum oder Zeitfrequenzraum durchgeführt zu werden, welche die Ausmessung einer grösseren Länge des Prüfguts voraussetzt und viel Zeit sowie Rechenleistung beansprucht.

Bei den statistischen Untersuchungen innerhalb einer jeden Menge können verschiedene Methoden zum Einsatz kommen. Eine davon ist die aus dem Stand der Technik bekannte, oben beschriebene Pearl-chain-Methode, die nun jedoch erfindungsgemässe in jeder der Mengen separat angewendet werden muss. Gemäss einer alternativen Methode werden Entfernungen von auf dem Prüfgut benachbarten Ereignissen aus der betreffenden Menge ermittelt. Die zur betreffenden Menge gehörenden Entfernungen werden miteinander verglichen. Auf ein regelmässiges Auftreten der Ereignisse in der betreffenden Menge wird dann geschlossen, wenn die Entfernungen innerhalb einer vorgegebenen Toleranz gleich sind. Dabei kann in der betreffenden Menge eine Periode aus den ähnlichen Entfernungen berechnet werden, z. B. als ein Mittelwert der ähnlichen Entfernungen. Ein Vorteil dieser zweiten Methode gegenüber der ersten Methode besteht also darin, dass in jeder Menge eine allfällige Periode ermittelt und ausgegeben werden kann. Die zweite Methode kann erweitert werden, indem in mindestens einer der Mengen mit entsprechenden statistischen Verfahren nicht nur eine Periode, sondern allenfalls mehr als eine Periode gesucht und allenfalls ausgegeben wird. Für die statistischen Untersuchungen kann eine maximale Anzahl regelmässiger Ereignisse vorgegeben werden, deren Erreichen als Kriterium für die Feststellung von regelmässigen Ereignissen im Prüfgut dient.

Nach einer Feststellung von regelmässigen Ereignissen kann derjenige Abschnitt des Prüfguts, der die regelmässigen Ereignisse enthält, aus dem Prüfgut entfernt werden.

Die erfindungsgemässe Vorrichtung zur Prüfung von regelmässigen Ereignissen in einem länglichen textilen Prüfgut beinhaltet einen Sensor zur Erfassung von Messwerten einer Eigenschaft des textilen Prüfguts entlang der Längsrichtung des textilen Prüfguts. Ferner beinhaltet sie eine mit dem Sensor verbundene Auswerteeinheit, welche für die Ermittlung von Werten mindestens eines Parameters des textilen Prüfguts aus den Messwerten eingerichtet ist. Ausserdem ist die Auswerteeinheit für die Definition von mindestens zwei verschiedenen, zusammenhängenden Punktmengen in einem Parameterraum, der durch den mindestens einen Parameter aufgespannt wird, und für die Bildung von mindestens zwei verschiedenen, mehr als ein Ereignis enthaltenden Mengen von Ereignissen im Prüfgut mit von einem Sollwert abweichenden Parameterwerten durch die Zuordnung von im Prüfgut festgestellten Ereignissen zu einer der Punktmengen gemäss den Parameterwerten der Ereignisse eingerichtet. Die Auswerteeinheit ist auch für die statistischen Untersuchungen der Ereignisse in den Mengen von Ereignissen und für die separate Ausgabe von Resultaten der statistischen Untersuchungen für jede der mindestens zwei Mengen eingerichtet. Ferner ist die Auswerteeinheit für die separate Durchführung von statistischen Untersuchungen der Ereignisse innerhalb der einzelnen, voneinander verschiedenen Mengen von Ereignissen in Bezug auf ein regelmässiges Auftreten eingerichtet.

Die Vorrichtung kann ferner eine mit der Auswerteeinheit verbundene Steuereinheit beinhalten. Die Steuereinheit enthält eine Speichereinheit zur Speicherung eines Ereignisfeldes, das einen Quadranten oder einen Teil eines Quadranten eines zweidimensionalen kartesischen Koordinatensystems beinhaltet, dessen Abszisse eine Länge von Ereignissen in der Längsrichtung und dessen Ordinate eine Querdimension oder eine Masse pro Längeneinheit des Prüfguts angibt. Ausserdem enthält die Steuereinheit eine Ausgabeeinheit zur Ausgabe des Ereignisfeldes sowie eine Eingabeeinheit zur Eingabe von Vorgaben für die statistischen Untersuchungen.

Die Ausgabeeinheit ist vorzugsweise als Bildschirm ausgebildet. Sie kann zusammen mit der Eingabeeinheit als Sensorbildschirm realisiert sein. Die Steuereinheit ist mit Vorteil in einer Textilverarbeitungsmaschine, bspw. einer Spinn- oder Spulmaschine, eingebaut. Die Vorrichtung kann eine mit der Auswerteeinheit verbundene Schneideinrichtung zur Entfernung eines Abschnitts des textilen Prüfguts, der die regelmässigen Ereignisse enthält, aus dem textilen Prüfgut beinhalten.

Dank der Erfindung können regelmässige Ereignisse differenzierter als im Stand der Technik beurteilt und festgestellt werden. Es wird vermieden, dass seltene, aber regelmässige Ereignisse nicht festgestellt werden, weil sie von häufigen, möglicherweise stochastisch verteilten Ereignissen überlagert werden. Vorkenntnisse über die zu untersuchenden Ereignisse sind praktisch keine nötig. Die Prüfung von regelmässigen Ereignissen kann während der Erzeugung oder Bearbeitung des Prüfguts, bspw. auf einem Garnreiniger während des Umspulens von Garn, erfolgen.

Ein weiterer Vorteil der Erfindung besteht darin, dass sie relativ regelmässige, aber nicht nur streng periodische Ereignisse prüfen kann. Diese Eigenschaft kann z. B. bei der Prüfung von Effektgarn nützlich sein, falls dessen Effekte eine gewisse Regelmässigkeit aufweisen. Die Erfindung kann nicht nur für die Prüfung des Vorhandenseins von unerwünschten regelmässigen Fehlstellen, sondern auch des Nichtvorhandenseins von erwünschten Effekten angewendet werden. Die Lagen der Effektpopulationen im Ereignisfeld sind oft bekannt; siehe z. B. die WO-2007/056883 A2. Die mindestens zwei Punktmengen können so gewählt werden, dass sie mit den erwarteten Lagen der Effektpopulationen übereinstimmen. Zeigt nun die erfindungsgemässe Prüfung an, dass in einer der Mengen keine regelmässigen Ereignisse vorliegen, so ist dies ein Hinweis darauf, dass die betreffenden Effekte fehlen oder zumindest nicht in der erwünschten Häufigkeit auftreten. In diesem Fall kann der Herstellungsprozess des Effektgarns entsprechend geändert werden.

AUFZÄHLUNG DER ZEICHNUNGEN

Nachfolgend wird die Erfindung anhand der schematischen Zeichnungen detailliert erläutert. Die Ausführungsbeispiele beziehen sich ohne Einschränkung der Allgemeinheit auf einen Garnreiniger an der Spulstelle einer Spulmaschine.

Figur 1     zeigt schematisch eine Spulmaschine mit einem Gamreinigersystem.
Figur 2     zeigt ein Ereignisfeld mit einer Reinigungskurve und zwei Ereignismengen gemäss der Erfindung.
Figur 3     zeigt schematisch ein Garn mit zwei Arten von Garnfehlern sowie je ein Verfahren zur Prüfung von regelmässigen Fehlstellen gemäss dem Stand der Technik und gemäss der Erfindung.
Figur 4     zeigt anhand des Garns von Figur 3 eine weitere Ausführungsform des erfindungsgemässen Verfahrens zur Prüfung von regelmässigen Fehlstellen.
Figur 5     zeigt ein weiteres Ereignisfeld, wie es in der Erfindung verwendet werden kann.

AUSFÜHRUNG DER ERFINDUNG

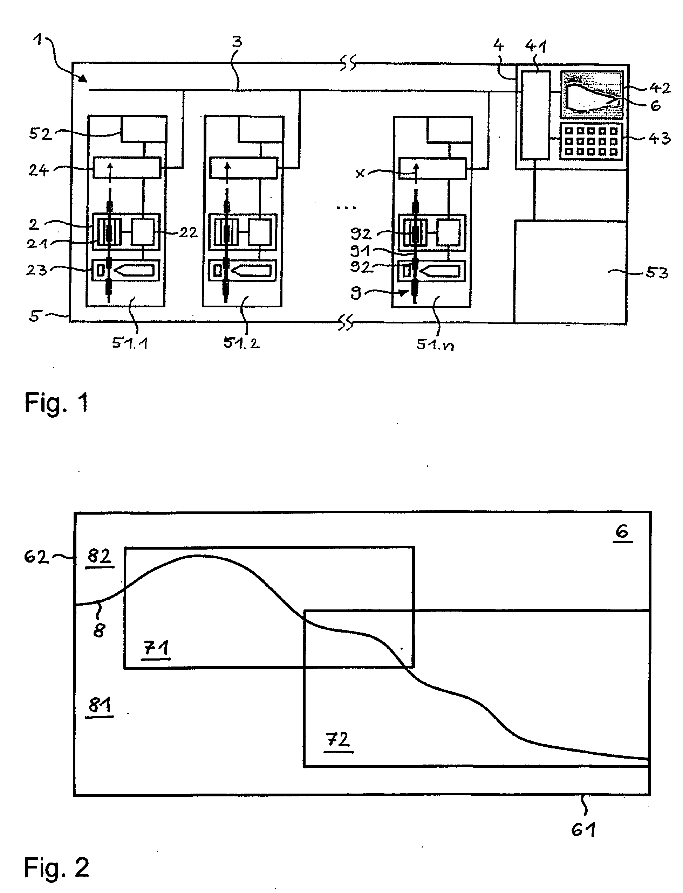

In **Figur 1** ist sehr schematisch eine erfindungsgemässe Spulmaschine 5 mit mehreren Spulstellen 51.1, 51.2, ..., 51.n dargestellt. Eine erfindungsgemässe Vorrichtung 1 ist in die Spulmaschine 5 eingebaut. An jeder Spulstelle 51.1, 51.2, ..., 52.n wird während des Umspulvorgangs Garn 9 von einem Messkopf 2 der erfindungsgemässen Vorrichtung 1 überwacht.

Der Messkopf 2 beinhaltet eine Abtasteinheit 21 zum Abtasten eines in Längsrichtung x bewegten Garns 9 mit fehlerfreien Abschnitten 91 und Fehlstellen oder Gamereignissen 92. Unter dem Begriff "Abtasten" wird hier die sequenzielle Aufnahme einer Vielzahl von Messwerten an verschiedenen, vorzugsweise äquidistanten Stellen des Garns 9 verstanden. Derartige Abtasteinheiten 21 sind an sich bekannt und brauchen hier deshalb nicht näher erläutert zu werden. Die Abtasteinheit 21 kann mit einem kapazitiven, optischen oder anderen Sensor ausgerüstet sein; es können auch mehrere gleiche oder verschiedene Sensoren innerhalb der Abtasteinheit 21 angeordnet sein. Die Abtasteinheit 21 kann mit Auswertemitteln für eine Vorauswertung der Messdaten ausgestattet sein. Sie gibt auf einer Datenleitung ein vorzugsweise elektrisches Ausgangssignal aus, das ein Mass für die Masse, die Dicke oder andere Eigenschaften des Garns 9 ist. Die Datenleitung führt zu einer Auswerteeinheit 22, die dazu eingerichtet ist, aus den Messwerten einen Garnparameter, z. B. die Garnmasse pro Längeneinheit, zu ermitteln. Zu diesem Zweck beinhaltet die Auswerteeinheit 22 geeignete analoge und/oder digitale Auswertemittel, z. B. einen Mikroprozessor oder einen digitalen Signalprozessor (digital signal processor, DSP). Sie kann auch weitere Mittel wie Speichermittel zum Speichern von Daten beinhalten. Die Auswerteeinheit 22 kann als eigenständige Baugruppe realisiert oder in einem gemeinsamen Messkopfgehäuse mit der Abtasteinheit 21, vorzugsweise auf einer einzigen Leiterplatte, integriert sein.

Vorzugsweise ist an jeder Spulstelle 51.1, 51.2, ..., 51.n auch eine Schneideinrichtung 23 angebracht. Mit dieser werden Garnabschnitte mit unzulässigen Fehlern aus dem Garn 9 herausgeschnitten. Die entsprechenden Schneidbefehle erhält die Schneideinrichtung 23 von der Auswerteeinheit 22. Die Schneideinrichtung 23 kann als eigenständige Baugruppe realisiert oder in einem gemeinsamen Messkopfgehäuse mit der Abtasteinheit 21 und /oder der Auswerteeinheit 22 untergebracht sein.

Der Messkopf 2 ist über einen Schnittstellenwandler 24 mit einer zentralen Steuereinheit 4 der erfindungsgemässen Vorrichtung 1 verbunden. Über die Verbindung wird der Messkopf 2 von der Steuereinheit 4 eingestellt und gesteuert, und der Messkopf 2 übermittelt Daten wie die ermittelten Garnparameter an die Steuereinheit 4. Eine Verbindungsleitung 3 zwischen allen Schnittstellenwandlern 24 und der Steuereinheit 4 kann als serieller Bus wie z. B. RS-485 ausgebildet sein. Der Schnittstellenwandler 24 kann zusätzlich auch mit einem Spulstellenrechner 52 der Spulstelle 51.1, 51.2, ..., 51.n verbunden sein. Die Steuereinheit 4 ist mit einem Zentralrechner 41 ausgestattet, welcher einerseits der Sammlung und weiteren Verarbeitung der von den Messköpfen 2 stammenden Messdaten, andererseits der Einstellung der Messköpfe 2 dient. Ferner weist die Steuereinheit 4 eine Ausgabeeinheit 42 und eine Eingabeeinheit 43 für eine Bedienungsperson auf. Die Ausgabeeinheit 42 dient der Ausgabe von Messdaten, von Resultaten der Auswertung und/oder der Veranschaulichung von eingegebenen Daten. Sie kann z. B. als Bildschirm und/oder Drucker ausgebildet sein. Die Eingabeeinheit 43 dient zum Eingeben von Daten durch die Bedienungsperson und insbesondere zur Eingabe und/oder zur Bearbeitung von (weiter unten erklärten) Mengen in einem Ereignisfeld 6, in denen separate statistische Untersuchungen von regelmässigen Ereignissen im Garn 9 durchgeführt werden. Die Eingabeeinheit 43 kann z. B. eine Tastatur oder eine Computermaus beinhalten. Die Ausgabeeinheit 42 und die Eingabeeinheit 43 können gemeinsam als Sensorbildschirm (Touchscreen) ausgebildet sein. Die Ausgabeeinheit 42 und die Eingabeeinheit 43 sind mittels Datenleitungen mit dem Zentralrechner 41 verbunden. Es kann somit gesagt werden, dass die Auswerteeinheit 22 zumindest indirekt ebenfalls mit der Ausgabeeinheit 42 und der Eingabeeinheit 43 verbunden ist.

Die Steuereinheit 4 ist mit einem Steuerrechner 53 der Spulmaschine 5 verbunden. Statt in der Steuereinheit 4 können der Zentralrechner 41, die Ausgabeeinheit 42 und/oder die Eingabeeinheit 43 in der Spulmaschine 5, z. B. im Steuerrechner 53, eingebaut sein. Die Datenleitungen können als Kabel oder drahtlos realisiert sein.

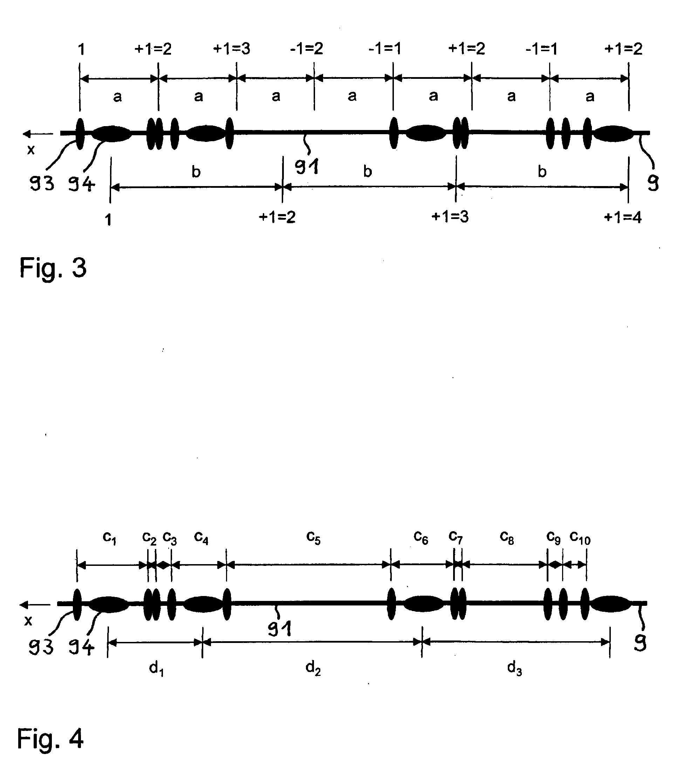

Auf der Ausgabeeinheit 42 können unter anderem die Garnereignisse in einem Ereignisfeld 6 dargestellt werden, wie es in **Figur 2** gezeigt ist. Das Ereignisfeld 6 ist ein zweidimensionales kartesisches Koordinatensystem. Längs einer Ordinate 62 werden Werte für positive Abweichungen eines Garnparameters, z. B. der Garnmasse pro Längeneinheit, von einem Sollwert und längs einer Abszisse 61 die Erstreckung oder Ausdehnung in Längsrichtung x oder Länge von Garnereignissen 92 (siehe Figur 1) aufgetragen. Der Gamparameter kann z. B. eine Garnmasse pro Längeneinheit oder ein Garndurchmesser sein. Die Garnparameterabweichungen werden üblicherweise in Prozenten angegeben, wobei der Sollwert den Wert 0 % hat; andere, z. B. absolute Einheiten sind möglich. Die Abszisse 61 gibt an, über welche Länge sich ein bestimmtes Gamereignis 92 oder eine bestimmte Garnparameterabweichung erstreckt. Es lässt sich somit sagen, dass das Ereignisfeld 6 ein durch die zwei Parameter Massenabweichung und Ereignislänge aufgespannter Parameterraum ist. In diesem Ereignisfeld 6 können Garnereignisse 92, d. h. gemessene Garnparameterabweichungen und ihre Längen, als Punkte eingetragen werden. Bei vielen Ereignissen 92 ergibt sich so eine Punktewolke (scatter plot), wie sie in Figur 4 dargestellt ist. Ein solches Ereignisfeld 6 kann bei Bedarf für eine Vielzahl von Garnreinigermessköpfen 2 ausgegeben, z. B. auf dem Bildschirm 42 angezeigt, werden.

Zusätzlich ist im Ereignisfeld 6 von Figur 2 eine Reinigungsgrenze in Form einer Reinigungskurve 8 eingetragen. Die Reinigungskurve 8 teilt das Ereignisfeld 6 in zwei Bereiche 81, 82 auf: einen ersten Bereich 81, in dem Abweichungen toleriert werden, und einen zweiten Bereich 82, der zum ersten Bereich 81 komplementär ist und in dem Abweichungen herausgeschnitten oder zumindest als unzulässige Fehler registriert werden. Die Reinigungskurve 8 gibt also an, wie weit ein Ereignis 92 gegebener Länge vom Sollwert abweichen darf, bzw. wie lang ein Ereignis 92 mit gegebener Abweichung sein darf, um gerade noch toleriert zu werden.

Erfindungsgemäss werden nun zur Prüfung von regelmässigen Ereignissen im Garn 9 mindestens zwei verschiedene Punktmengen 71, 72 von Gamereignissen 92 vorgegeben. Jede der Punktmengen für sich ist zusammenhängend im Sinne der mathematischen Topologie. Die im Garn festgestellten Garnereignisse 92 werden gemäss ihren gemessenen Parameterwerten einer der Punktmengen 71, 72 zugeordnet. Die Garnereignisse 92 werden dann statistisch in Bezug auf ein regelmässiges Auftreten in jeder der Punktmengen 71, 72 separat untersucht. Die statistischen Untersuchungen können in der Auswerteeinheit 22 des Messkopfes 2 erfolgen. Im Ausführungsbeispiel von Figur 2 haben die beiden Punktmengen 71, 72 die Form von Rechtecken, die sich teilweise überlappen. Andere Formen sind jedoch möglich, und eine Überlappung ist nicht nötig. Jede der Mengen 71, 72 sollte zumindest teilweise den Bereich 81 der zulässigen Garnereignisse unterhalb der Reinigungskurve 8 überdecken. Nur in diesem unteren Bereich 81 findet ja die Überprüfung auf regelmässige Fehlstellen hin statt; im oberen Bereich 82 sind ohnehin alle Garnereignisse unzulässig.

Anhand der **Figur 3** soll ein Vorteil des erfindungsgemässen Verfahrens gegenüber dem Stand der Technik verdeutlicht werden. Die Figur zeigt in der Mitte schematisch ein Garn 9 mit zwei Arten von Gamereignissen 93, 94:

- kurzen, dicken Ereignissen 93, die entlang der Gamlängsrichtung x häufig auftreten und stochastisch verteilt sind, und
- langen, weniger dicken Ereignissen 94, die entlang der Gamlängsrichtung x selten auftreten und ziemlich regelmässig verteilt sind.

Oberhalb des Garns 9 ist angedeutet, wie der Pearl-Chain-Kanal gemäss Kapitel 6 "Periodic yarn faults" des USTER® QUANTUM 2 Application Manual Winding, Uster Technologies AG, November 2006, mit diesem Garn 9 verfahren würde. Gemäss diesem Stand der Technik werden alle Garnereignisse 93, 94 gleich behandelt, d. h. es wird nicht zwischen den beiden Typen 93, 94 von Garnereignissen unterschieden. Dem Pearl-chain-Kanal wird ein Fehlerabstand a vorgegeben. Sobald ein erstes Garnereignis 93 eintritt, wird ein laufender Zähler auf 1 gesetzt. Wenn es innerhalb des Fehlerabstands a zu einem weiteren Ereignis 94 kommt, wird der laufende Zähler um eins erhöht; wenn nicht, wird der laufende Zähler um 1 vermindert. Sobald der laufende Zähler grösser ist als eine vorgegebene Höchstzahl von Gamereignissen, so liegt ein Pearl Chain vor, und ein Garnschnitt wird ausgelöst. Wie das Beispiel von Figur 3 zeigt, erreicht der laufende Zähler kaum einen hohen Wert, weil immer wieder Garnabschnitte ohne Fehler 93, 94 vorkommen, die den Zählerstand vermindern. Die häufigen kurzen, dicken Garnereignisse 93 überlagern die seltenen langen, weniger dicken Garnereignisse 94, so dass die Regelmässigkeit der letzteren nicht festgestellt werden kann. Dabei verursachen die langen, weniger dicken Garnereignisse 94 meist grössere Störungen im Bild des Gewebes oder Gestricks als die kurzen, dicken Ereignisse 93.

Im Gegensatz zum Stand der Technik berücksichtigt das erfindungsgemässe Verfahren das Vorhandensein von Gamereignissen 93, 94 mit unterschiedlichen Eigenschaften. Es kann z. B. zwischen den beiden Typen von Gamereignissen 93, 94 unterscheiden, wie sie in Figur 3 schematisch eingezeichnet sind. Die Unterscheidung erfolgt vorzugsweise mittels der beiden in Figur 2 veranschaulichten Punktmengen 71, 72. Die kurzen, dicken Garnereignisse 93 werden der ersten Punktmenge 71 zugeordnet, die langen, weniger dicken Garnereignisse 94 der zweite Punktmenge 72. Durch diese Zuordnungen werden zwei verschiedene Mengen von Garnereignissen 93, 94 gebildet, die nachfolgend der Einfachheit halber ebenfalls mit den Bezugszeichen 71 bzw. 72 bezeichnet werden. Die statistische Untersuchung der Regelmässigkeit von Garnereignissen 93, 94 erfolgt in jeder der beiden Mengen 71, 72 separat.

In einer ersten Ausführungsform des erfindungsgemässen Verfahrens werden Fehlerabstände a bzw. b vorgegeben, die für die beiden Mengen 71, 72 unterschiedlich, innerhalb einer jeden Menge 71, 72 jedoch konstant sind. Wird ein Gamereignis 93, 94 festgestellt, so wird es zunächst in eine der beiden Mengen 71, 72 eingeteilt. In Figur 3 ist oberhalb des Garns 9 der laufende Zähler für die erste Menge 71, unterhalb des Garns 9 für die zweite Menge 72 illustriert. Der erste laufende Zähler stellt keine Regelmässigkeit von Gamereignissen 93 der ersten Menge 71 fest. Dagegen kommt der zweite laufende Zähler schnell auf einen hohen Wert, der eine Regelmässigkeit von Gamereignissen 94 in der zweiten Menge 72 hindeutet. Somit ist das erfindungsgemässe Verfahren, im Gegensatz zum Stand der Technik, in der Lage, die Regelmässigkeit der langen, weniger dicken Ereignisse 94 festzustellen.

Eine zweite Methode der statistischen Untersuchung der Regelmässigkeit von Garnereignissen, wie sie im erfindungsgemässen Verfahren zur Anwendung kommen kann, ist in **Figur 4** illustriert. Auch hier wird jedes festgestellte Garnereignis 93, 94 zunächst in eine der beiden Mengen 71, 72 eingeteilt. Es wird jeweils die Entfernung zum vorhergehenden Gamereignis 93, 94 aus derselben Menge 71, 72 ermittelt; in Figur 4 sind diese Distanzen mit $c_1$, $c_2$,... für die kurzen, dicken Garnereignisse 93 aus der ersten Menge 71 und mit $d_1$, $d_2$,... für die langen, weniger dicken Garnereignisse 94 aus der zweiten Menge 72 bezeichnet. Die zur selben Menge 71 bzw. 72 gehörenden Entfernungen $c_1$, $c_2$,... bzw. $d_1$, $d_2$,... werden miteinander verglichen. Wenn sie innerhalb einer vorgegebenen Toleranz gleich sind, liegt in der jeweiligen Menge 71 bzw. 72 ein regelmässiges Auftreten von Gamereignissen 93 bzw. 94 vor; unterscheiden sie sich deutlich voneinander, so gibt es keine Regelmässigkeit. Im Beispiel von Figur 4 ist eindeutig

$$c_1 \neq c_2 \neq \ldots \neq c_{10} \,,$$

so dass die Untersuchung auf regelmässige Ereignisse hin in der ersten Menge 71 negativ ausfällt; mit anderen Worten: Die kurzen, dicken Garnereignisse 93 sind stochastisch verteilt. Hingegen gilt innerhalb einer gewissen, vorgebbaren Toleranz:

$$d_1 \approx d_2 \approx d_3 \,,$$

was auf eine gewisse Regelmässigkeit der langen, weniger dicken Garnereignisse 94 der zweiten Menge 72 hindeutet. Es kann eine Höchstzahl von Gamereignissen 94 mit ähnlichen Entfernungen $d_1 \approx d_2 \approx d_3$ vorgegeben werden, die überschritten werden muss, damit auf das Vorliegen eines regelmässigen Auftretens von Gamereignissen 94 geschlossen wird. Bei Überschreiten der Höchstzahl kann in der betreffenden Menge 72 eine Periode d berechnet werden, z. B. als Mittelwert der ähnlichen Entfernungen $d_1 \approx d_2 \approx d_3$:

$$d = (d_1 + d_2 + d_3)/3 \,.$$

Die Periode d kann auf der Ausgabeeinheit 42 der zentralen Steuereinheit 4 (siehe Figur 1) ausgegeben werden.

Die obige Untersuchungsmethode ist ein bewusst einfaches Beispiel für eine statistische Untersuchung. Alternativ oder zusätzlich können andere, komplexere Methoden zum Einsatz kommen. So könnte etwa die Häufigkeitsverteilung der innerhalb der betreffenden Menge 72 gemessenen Distanzen $d_1$, $d_2$,... betrachtet und daraus unter Umständen auf das Vorhandensein von zwei verschiedenen Perioden geschlossen werden.

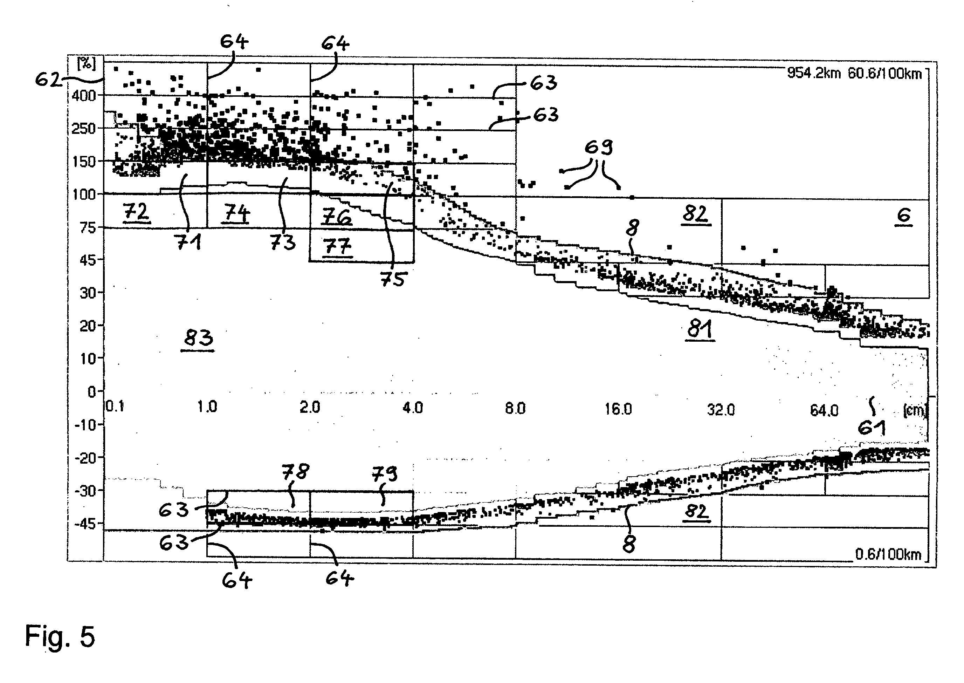

**Figur 5** zeigt, in einer zu Figur 2 analogen Darstellung, eine erweiterte Darstellung eines Ereignisfeldes 6, wie sie auf der Ausgabeeinheit 42 der zentralen Steuereinheit 4 (siehe Figur 1) ausgegeben werden kann. Hier beinhaltet das Ereignisfeld 6 nicht nur den ersten, sondern zusätzlich auch noch den vierten Quadranten. Die Längenachsen 61 der beiden Quadranten fallen zusammen. Im ersten Quadranten werden durch lokale Massenzunahme verursachte Dickstellen, im vierten Quadranten durch lokale Massenabnahme verursachte Dünnstellen gegenüber der jeweiligen Ereignislänge aufgetragen. Eine ermittelte Abweichung und ihre Länge bilden zusammen die Koordinaten eines Garnereignisses 92 (siehe Figur 1), das einen Punkt 69 im Ereignisfeld 6 definiert und dort bspw. mit einem quadratischen Symbol dargestellt werden kann. Alle Ereignisse 92 bilden zusammen eine so genannte Punktewolke (englisch scatter plot). Um die Längenachse 61 herum befindet sich ein Bereich 83, der "Garnkörper" genannt wird und in Figur 5 grau eingezeichnet ist. Er zeichnet sich durch eine sehr hohe Dichte an Gamereignissen 92 aus, die somit "zum Garn 9 gehören", nicht dargestellt und schon gar nicht ausgereinigt zu werden brauchen. Der Garnkörper 83 wird vorzugsweise durch Dichtekurven, d. h. Linien konstanter Ereignisdichte, begrenzt. Er ist bezüglich der Längenachse 61 nicht notwendigerweise symmetrisch. Die Achsen 61, 62 für Länge und Massenzunahme bzw. Massenabnahme sind vorzugsweise logarithmisch, fast logarithmisch oder teilweise logarithmisch eingeteilt. Im Ereignisfeld 6 von Figur 5 ist auch eine Reinigungskurve 8 eingezeichnet. Die Reinigungskurve 8 kann, muss aber nicht einer Dichtelinie folgen.

Zumindest ein Teil des Ereignisfeldes 6 ist durch waagrechte Klassengrenzen 63 und senkrechte Klassengrenzen 64 in rechteckige Klassen 71-79 für Garnereignisse 92 unterteilt. Solche Klassen 71-79 sind aus dem Stand der Technik bekannt und dienten ursprünglich der besseren statistischen Erfassung ähnlicher Ereignisse 92. So konnten z. B. die festgestellten Ereignisse 92 in jeder Klasse 71-79 gezählt und die Resultate klassenweise ausgegeben werden. Gemäss der vorliegenden Erfindung ist es als vorteilhaft erkannt worden, mittels dieser Klassen 71-79 die Punktmengen zu definieren, in denen die statistische Untersuchung der regelmässigen Garnereignisse separat durchgeführt wird.

Selbstverständlich ist die vorliegende Erfindung nicht auf die oben diskutierten Ausführungsformen beschränkt. Insbesondere kann die statistische Untersuchung der Garnereignisse auf ein regelmässiges Auftreten hin mit einer anderen als der oben beschriebenen Methode erfolgen. Bei Kenntnis der Erfindung wird der Fachmann weitere Varianten herleiten können, die auch zum Gegenstand der vorliegenden Erfindung gehören, wie sie in den unabhängigen Patentansprüchen definiert ist.

BEZUGSZEICHENLISTE

1    Vorrichtung

2    Messkopf
21    Abtasteinheit
22    Auswerteeinheit
23    Schneideinrichtung

3    Datenleitung

4    Steuereinheit
41    Zentralrechner
42    Ausgabeeinheit
43    Eingabeeinheit

5    Spulmaschine
51.1, 51.2, ..., 51.n    Spulstellen
52    Spulstellenrechner
53    Steuerrechner

6    Ereignisfeld
61    Abszisse
62    Ordinate
63    waagrechte Klassengrenze
64    senkrechte Klassengrenze
69    Ereignispunkte

71-79    Punktmengen bzw. Ereignismengen

8    Reinigungsgrenze

81    Bereich von zulässigen Ereignissen
82    Bereich von unzulässigen Ereignissen
83    Garnkörper

9     Garn
91    fehlerfreier Garnabschnitt
92    Garnereignisse
93    kurze, dicke Garnereignisse
94    lange, weniger dicke Garnereignisse

a, b   vorgegebene Fehlerabstände
c, d   ermittelte Ereignisentfernungen
x      Längs- und Bewegungsrichtung des Garns

**Patentansprüche**

**1.** Verfahren zur Prüfung von regelmässigen Ereignissen in einem länglichen textilen Prüfgut (9), wobei
Messwerte einer Eigenschaft des Prüfguts (9) entlang der Längsrichtung (x) des Prüfguts (9) erfasst werden,
Werte mindestens eines Parameters des Prüfguts (9) aus den Messwerten ermittelt werden,
mindestens zwei verschiedene, zusammenhängende Punktmengen (71-79) in einem Parameterraum, der durch den mindestens einen Parameter aufgespannt wird, definiert werden,
mindestens zwei verschiedene, mehr als ein Ereignis (92-94) enthaltende Mengen von Ereignissen (92-94) im Prüfgut (9) mit von einem Sollwert abweichenden Parameterwerten gebildet werden, indem im Prüfgut (9) festgestellte Ereignisse (92-94) gemäss ihren Parameterwerten einer der Punktmengen (71-79) zugeordnet werden,
die Ereignisse (92-94) innerhalb der einzelnen, voneinander verschiedenen Mengen von Ereignissen (92-94) in Bezug auf ein regelmässiges Auftreten separat statistisch untersucht werden und
Resultate der statistischen Untersuchungen für jede der mindestens zwei Mengen von Ereignissen (92-94) separat ausgegeben werden.

**2.** Verfahren nach Anspruch 1, wobei die Punktmengen (71-79) als zusammenhängende Flächen in einem zweidimensionalen Ereignisfeld (6) definiert werden.

**3.** Verfahren nach einem der vorangehenden Ansprüche, wobei der Parameterraum durch eine Querdimension des Prüfguts (9), durch eine Masse pro Längeneinheit des Prüfguts (9) und/oder durch eine Länge von Ereignissen in der Längsrichtung (x) aufgespannt wird.

**4.** Verfahren nach den Ansprüchen 2 und 3, wobei das Ereignisfeld (6) einen Quadranten oder einen Teil eines Quadranten eines zweidimensionalen kartesischen Koordinatensystems beinhaltet, dessen Abszisse (61) eine Länge von Ereignissen in der Längsrichtung (x) und dessen Ordinate (62) eine Querdimension oder eine Masse pro Längeneinheit des Prüfguts (9) angibt.

**5.** Verfahren nach Anspruch 4, wobei die Punktmengen (71-79) rechteckige Klassen für Ereignisse (92-94) sind, die durch eine Unterteilung zumindest eines Teils des Ereignisfeldes (6) durch waagrechte Klassengrenzen (63) und senkrechte Klassengrenzen (64) gebildet werden.

**6.** Verfahren nach einem der vorangehenden Ansprüche, wobei die statistischen Untersuchungen ausschliesslich im Ortsraum oder im Zeitraum erfolgen.

**7.** Verfahren nach Anspruch 6, wobei die statistische Untersuchung in jeder der mindestens zwei Mengen von Ereignissen (92-94) durchgeführt wird, indem, Entfernungen ($d_1$, $d_2$, $d_3$) von auf dem Prüfgut (9) benachbarten Ereignissen (94) aus der betreffenden Menge (72) ermittelt werden,
die zur betreffenden Menge (72) gehörenden Entfernungen ($d_1$, $d_2$, $d_3$) miteinander verglichen werden, und
auf ein regelmässiges Auftreten der Ereignisse (94) in der betreffenden Menge (72) geschlossen wird, wenn die Entfernungen ($d_1$, $d_2$, $d_3$) innerhalb einer vorgegebenen Toleranz gleich sind.

**8.** Verfahren nach Anspruch 7, wobei in der betreffenden Menge (72) eine Periode (d) aus den ähnlichen Entfernungen ($d_1$, $d_2$, $d_3$) berechnet wird, z. B. als ein Mittelwert der ähnlichen Entfernungen ($d_1$, $d_2$, $d_3$).

9. Verfahren nach einem der vorangehenden Ansprüche, wobei für die statistischen Untersuchungen eine maximale Anzahl regelmässiger Ereignisse vorgegeben wird, deren Erreichen als Kriterium für die Feststellung von regelmässigen Ereignissen im Prüfgut (9) dient.

10. Verfahren nach einem der vorangehenden Ansprüche, wobei nach einer Feststellung von regelmässigen Ereignissen derjenige Abschnitt des Prüfguts (9), der die regelmässigen Ereignisse enthält, aus dem Prüfgut (9) entfernt wird.

11. Vorrichtung (1) zur Prüfung von regelmässigen Ereignissen in einem länglichen textilen Prüfgut (9), beinhaltend einen Sensor (21) zur Erfassung von Messwerten einer Eigenschaft des Prüfguts (9) entlang der Längsrichtung (x) des Prüfguts (9) sowie
eine mit dem Sensor (21) verbundene Auswerteeinheit (22), welche
für die Ermittlung von Werten mindestens eines Parameters des Prüfguts (9) aus den Messwerten,
für die Definition von mindestens zwei verschiedenen, zusammenhängenden Punktmengen (71-79) in einem Parameterraum, der durch den mindestens einen Parameter aufgespannt wird,
für die Bildung von mindestens zwei verschiedenen, mehr als ein Ereignis (92-94) enthaltenden Mengen (71-79) von Ereignissen (92-94) im Prüfgut (9) mit von einem Sollwert abweichenden Parameterwerten durch die Zuordnung von im Prüfgut (9) festgestellten Ereignissen (92-94) zu einer der Punktmengen (71-79) gemäss den Parameterwerten der Ereignisse (92-94),
für die statistischen Untersuchungen der Ereignisse (92-94) in den Mengen (71-79) von Ereignissen (92-94) und
für die separate Ausgabe von Resultaten der statistischen Untersuchungen für jede der mindestens zwei Mengen eingerichtet ist,
**dadurch gekennzeichnet,**
**dass** die Auswerteeinheit (22)
für die separate Durchführung von statistischen Untersuchungen der Ereignisse (92-94) innerhalb der einzelnen, voneinander verschiedenen Mengen von Ereignissen (92-94) in Bezug auf ein regelmässiges Auftreten eingerichtet ist.

12. Vorrichtung (1) nach Anspruch 11, ferner beinhaltend eine mit der Auswerteeinheit (22) verbundene Steuereinheit (4) mit
einer Speichereinheit zur Speicherung eines Ereignisfeldes (6), das einen Quadranten oder einen Teil eines Quadranten eines zweidimensionalen kartesischen Koordinatensystems beinhaltet, dessen Abszisse (61) eine Länge von Ereignissen in der Längsrichtung (x) und dessen Ordinate (62) eine Querdimension oder eine Masse pro Längeneinheit des Prüfguts (9) angibt,
einer Ausgabeeinheit (42) zur Ausgabe des Ereignisfeldes (6) sowie einer Eingabeeinheit (43) zur Eingabe von Vorgaben für die statistischen Untersuchungen.

13. Vorrichtung (1) nach Anspruch 12, wobei die Steuereinheit (4) in einer Textilverarbeitungsmaschine (5), bspw. einer Spinn- oder Spulmaschine, eingebaut ist.

14. Vorrichtung (1) nach einem der Ansprüche 11-13, wobei die Vorrichtung (1) eine mit der Auswerteeinheit (22) verbundene Schneideinrichtung (23) zur Entfernung eines Abschnitts des textilen Prüfguts (9), der die regelmässigen Ereignisse enthält, aus dem textilen Prüfgut (9) beinhaltet.

## Claims

1. A method for analyzing regular events in an elongated textile test material (9),
wherein
measured values of a property of the test material (9) are detected along the longitudinal direction (x) of the test material (9),
values of at least one parameter of the test material (9) are determined from the measured values,
at least two different connected point sets (71 to 79) are defined in a parameter space which is spanned by the at least one parameter,
at least two different sets of events (92 to 94) containing more than one event (92 to 94) in the test material (9) with parameter values deviating from a setpoint value are formed in that events (92 to 94) determined in the test material (9) are assigned to one of the point sets (71 to 79) according to their parameter values,
the events (92 to 94) within the individual sets of events (92 to 94), which differ from one another, are statistically analyzed separately with regard to a regular occurrence, and

results of the statistical analyses are output separately for each of the at least two sets of events (92 to 94).

2. The method according to claim 1, wherein the point sets (71 to 79) are defined as connected areas in a two-dimensional event field (6).

3. The method according to one of the preceding claims, wherein the parameter space is spanned by a transverse dimension of the test material (9), by a mass per unit of length of the test material (9) and/or by a length of events in the longitudinal direction (x).

4. The method according to claims 2 and 3, wherein the event field (6) contains a quadrant or a part of a quadrant of a two-dimensional Cartesian coordinate system, the abscissa (61) of which indicates a length of events in the longitudinal direction (x) and the ordinate (62) of which indicates a transverse dimension or a mass per unit of length of the test material (9).

5. The method according to claim 4, wherein the point sets (71 to 79) are rectangular classes for events (92 to 94) which are formed by horizontal class limits (63) and vertical class limits (64) by subdividing at least a part of the event field (6).

6. The method according to one of the preceding claims, wherein the statistical analyses occur exclusively in the spatial domain or in the time domain.

7. The method according to claim 6, wherein the statistical analysis is performed in each of the at least two sets of events (92 to 94), in that
distances ($d_1$, $d_2$, $d_3$) of events (94) in the respective set (72) which are adjacent to one another are determined ,
the distances ($d_1$, $d_2$, $d_3$) belonging to the respective set (72) are compared with one another, and
conclusions are drawn on a regular occurrence of the events (94) in the respective set (72) if the distances ($d_1$, $d_2$, $d_3$) are equal within a predetermined tolerance.

8. The method according to claim 7, wherein a period (d) is calculated from the similar distances ($d_1$, $d_2$, $d_3$) in the respective quantity (72), e.g. as a mean value of the similar distances ($d_1$, $d_2$, $d_3$).

9. The method according to one of the preceding claims, wherein a maximum number of regular events is predetermined for the statistical analyses, the achievement of which is used as a criterion for determining regular events in the test material (9).

10. The method according to one of the preceding claims, wherein after determination of regular events the section of the test material (9) which contains the regular events is removed from the test material (9).

11. An apparatus (1) for analyzing regular events in an elongated textile test material (9), containing
a sensor (21) for detecting measured values of a property of the test material (9) along the longitudinal direction (x) of the test material (9), and
an evaluation unit (22) which is connected with the sensor (21) and which is set up
for the determination of values of at least one parameter of the test material (9) from the measured values,
for the definition of at least two different connected point sets (71 to 79) in a parameter space which is spanned by the at least one parameter,
for the formation of at least two different sets of events (92 to 94) containing more than one event (92 to 94) in the test material (9) with parameter values deviating from a setpoint value by the assignment of events (92 to 94) determined in the test material (9) to one of the point sets (71 to 79) according to the parameter values of the events (92 to 94),
for the statistical analyses of the events (92 to 94) in the sets (71 to 79) of events (92 to 94), and
for the separate output of results of the statistical analyses for each of the at least two sets,
**characterized in that**
the evaluation unit (22) is set up
for the separate performance of statistical analyses of the events (92 to 94) within the individual sets of events (92 to 94), which differ from one another, with regard to a regular occurrence.

12. The apparatus (1) according to claim 11, further containing a control unit (4) connected with the evaluation unit (22), comprising

a memory unit for storing an event field (6) which contains a quadrant or part of a quadrant of a two-dimensional Cartesian coordinate system, the abscissa (61) of which indicates a length of events in the longitudinal direction (x) and the ordinate (62) of which indicates a transverse dimension or a mass per unit of length of the test material (9), an output unit (42) for the output of the event field (6), and an input unit (43) for the input of default values for the statistical analyses.

13. The apparatus (1) according to claim 12, wherein the control unit (4) is installed in a textile processing machine (5) such as a spinning or winding machine.

14. The apparatus (1) according to one of the claims 11 to 13, wherein the apparatus (1) contains a cutting device (23) connected with the evaluation unit (22) for removing from the textile test material (9) a section of the textile test material (9) which contains the regular events.

**Revendications**

1. Procédé pour contrôler des événements réguliers dans un article textile allongé à contrôler (9), dans lequel des valeurs de mesure d'une propriété de l'article à contrôler (9) sont relevées le long du sens longitudinal (x) de l'article à contrôler (9),
des valeurs d'au moins un paramètre de l'article à contrôler (9) sont déterminées à partir des valeurs de mesure,
au moins deux ensembles de points cohérents différents (71-79) sont définis dans un espace de paramètres passant par l'au moins un paramètre,
au moins deux ensembles d'événements (92-94) différents contenant plus d'un événement (92-94) sont formés dans l'article à contrôler (9) avec des valeurs de paramètres différentes de la valeur de consigne en associant des événements (92-94) observés dans l'article à contrôler (9) à l'un des ensembles de points (71-79) selon leurs valeurs de paramètres,
les événements (92-94) font l'objet d'une analyse statistique séparée concernant une apparition régulière dans chacun des ensembles d'événements (92-94) différents les uns des autres et
les résultats des analyses statistiques sont sortis séparément pour chacun des au moins deux ensembles d'événements (92-94).

2. Procédé selon la revendication 1, dans lequel les ensembles de points (71-79) sont définis comme des surfaces cohérentes dans un champ d'événements (6) à deux dimensions.

3. Procédé selon l'une des revendications précédentes, dans lequel l'espace de paramètres est défini par une dimension transversale de l'article à contrôler (9), par une masse par unité de longueur de l'article à contrôler (9) et/ou par une longueur d'événements dans l'orientation longitudinale (x).

4. Procédé selon les revendications 2 et 3, dans lequel le champ d'événements (6) contient un quadrant ou une partie d'un quadrant d'un système de coordonnées cartésien à deux dimensions, dont l'abscisse (61) indique une longueur des événements dans le sens longitudinal (x) et l'ordonnée (62) une dimension transversale ou une masse par unité de longueur de l'article à contrôler (9).

5. Procédé selon la revendication 4, dans lequel les ensembles de points (71-79) sont des classes rectangulaires pour les événements (92-94), qui sont formées par une subdivision d'au moins une partie du champ d'événements (6) par des limites de classe horizontales (63) et des limites de classe verticales (64).

6. Procédé selon l'une des revendications précédentes, dans lequel les analyses statistiques sont effectuées exclusivement dans l'espace local ou dans l'espace temporel.

7. Procédé selon la revendication 6, dans lequel l'analyse statistique est effectuée dans chacun des au moins deux ensembles d'événements (92-94) en déterminant des distances ($d_1$, $d_2$, $d_3$) d'événements (94) de l'ensemble (72) concerné voisins sur l'article à contrôler (9),
en comparant les distances ($d_1$, $d_2$, $d_3$) correspondant à l'ensemble (72) concerné entre elles et
en concluant à une apparition régulière des événements (94) dans l'ensemble (72) concerné si les distances ($d_1$, $d_2$, $d_3$) sont égales dans les limites d'une tolérance prédéfinie.

8. Procédé selon la revendication 7, dans lequel une période (d) est calculée dans l'ensemble (72) concerné à partir

des distances ($d_1$, $d_2$, $d_3$) similaires, par exemple sous la forme d'une moyenne des distances ($d_1$, $d_2$, $d_3$) similaires.

9. Procédé selon l'une des revendications précédentes, dans lequel est prédéfini pour les analyses statistiques un nombre maximal d'événements réguliers qui sert, lorsqu'il est atteint, de critère pour la constatation d'événements réguliers dans l'article à contrôler (9).

10. Procédé selon l'une des revendications précédentes dans lequel, après la constatation d'événements réguliers, la section de l'article à contrôler (9) qui contient les événements réguliers est enlevée de l'article à contrôler (9).

11. Dispositif (1) pour le contrôle d'événements réguliers dans un article textile allongé à contrôler (9), comprenant un capteur (21) pour relever des valeurs de mesure d'une propriété de l'article à contrôler (9) le long du sens longitudinal (x) de l'article à contrôler (9) et
une unité d'analyse (22) reliée au capteur (21), qui est conçue
pour déterminer des valeurs d'au moins un paramètre de l'article à contrôler (9) à partir des valeurs de mesure,
pour définir au moins deux ensembles de points (71-79) cohérents différents dans un espace de paramètres défini par l'au moins un paramètre,
pour créer au moins deux ensembles (71-79) d'événements (92-94) différents contenant plus d'un événement (92-94) dans l'article à contrôler (9) avec des valeurs de paramètres différentes d'une valeur de consigne en associant des événements (92-94) constatés dans l'article à contrôler (9) à un des ensembles de points (71-79) selon les valeurs de paramètre des événements (92-94), pour effectuer des analyses statistiques des événements (92-94) dans les ensembles (71-79) d'événements (92-94) et
pour émettre séparément en sortie des résultats des analyses statistiques pour chacun des au moins deux ensembles,
**caractérisé en ce que**
l'unité d'analyse (22) est conçue pour effectuer séparément des analyses statistiques des événements (92-94) concernant une apparition régulière dans chacun des ensembles d'événements (92-94) différents les uns des autres.

12. Dispositif (1) selon la revendication 11, contenant en outre une unité de commande (4) reliée à l'unité d'analyse (22) avec
une unité de mémoire pour mémoriser un champ d'événements (6) contenant un quadrant ou une partie d'un quadrant d'un système de coordonnées cartésien à deux dimensions, dont l'abscisse (61) indique une longueur des événements dans le sens longitudinal (x) et l'ordonnée (62) une dimension transversale ou une masse par unité de longueur de l'article à contrôler (9),
une unité de sortie (42) pour produire en sortie le champ d'événements (6) et
une unité d'entrée (43) pour introduire des instructions pour les analyses statistiques.

13. Dispositif (1) selon la revendication 12, dans lequel l'unité de commande (4) est intégrée dans une machine de transformation textile (5), par exemple un métier à filer ou une bobineuse.

14. Dispositif (1) selon l'une des revendications 11 à 13, lequel dispositif (1) comprend un dispositif de coupe (23) relié à l'unité d'analyse (22) pour enlever de l'article textile à contrôler (9) une section de l'article textile à contrôler (9) qui contient les événements réguliers.

Fig. 1

Fig. 2

Fig. 3

Fig. 4

Fig. 5

EP 2 564 189 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5537811 A
- EP 0924513 A1
- WO 9313407 A1
- US 2950435 A
- US 5592849 A
- EP 2090538 A2
- DE 3928417 A1
- WO 2007056883 A2

**In der Beschreibung aufgeführte Nicht-Patentliteratur**

- Periodic yarn faults. USTER® QUANTUM 2 Application Manual Winding. Uster Technologies AG, November 2006
- Classification values. USTER® QUANTUM 2 Application Manual Winding. Uster Technologies AG, November 2006